# EUROPEAN PATENT SPECIFICATION

(11) **EP 1 731 187 B1**
(45) Date of publication and mention of the grant of the patent: **23.07.2008**
(21) Application number: 06252947.4
(22) Date of filing: 07.06.2006
(51) Int. Cl.: A61M 16/00, G06K 19/07

(54) **Respiratory apparatus with a carbon dioxide absorber**
Beatmungsgerät mit einem Kohlendioxidabsorber
Appareil respiratoire avec un absorbeur de dioxide de carbone

(30) Priority: 10.06.2005 DE 102005026838
(43) Date of publication of application: 13.12.2006
(73) Proprietor: Dräger Medical AG & Co. KG, 23542 Lübeck (DE)
(72) Inventor: Schermeier, Olaf, 23560 Lübeck (DE); Lange, Jürgen-Ralf, 23611 Bad Schwartau (DE); Lokotsch, Heiko, 19205 Pokrent (DE)
(74) Representative: Strauss, Steffen

(56) References cited:
- EP-A- 0 769 304
- EP-A- 1 170 023
- WO-A-20/04033024
- WO-A-20/04040202
- WO-A-20/05118036
- US-A1- 2005 118 048

## Description

A medical apparatus with a base unit and items of ancillary equipment connectable to the base unit is known from DE 201 13 789 U1. The items of ancillary equipment are tube systems, containers and functional elements that are required for the operation of the medical apparatus. Each of the items of ancillary equipment is fitted with a transponder, which contains information concerning the type of ancillary equipment and the producer. This information is detected by a transponder-interrogator affixed to the base unit, whereby the possibility also exists of storing data on the memory chip of the transponder via the transponder-interrogator. By means of the transponders fitted on the items of ancillary equipment, the base unit knows at all times which items of ancillary equipment it is equipped with.

In medical apparatuses from the area of anaesthesia-respiration technology, a carbon dioxide absorber is required in the re-inhalation operation in order to remove the carbon dioxide exhaled by the patient from the respiratory gas. Carbon dioxide absorbers are offered here in various designs. In one group of absorbers, the absorber housing is opened and filled with respiratory lime as a bulk material. The respiratory lime is later disposed of after use and replaced by fresh respiratory lime. Disposable absorbers which are completely disposed of after use are also known.

In the case of the known carbon dioxide absorbers, depletion is noted by visual observation of the colour change of the respiratory lime. Since the borderline for the colour change often cannot be unequivocally identified, the depletion can thus only be estimated roughly. With the known devices, therefore, it is common practice to replace the absorber or the respiratory lime in the absorber at fixed intervals. As a result, however, the capacity of the respiratory lime is often only partially utilised, since the replacement interval is determined according to the most unfavourable mode of operation.

WO-A-2004/040202 discloses a device and process for the heating and humidification of gas, especially respiratory gas.

US-A-2005/1 18048 discloses a cartridge or cassette adapted to be attached to an apparatus for controlling the flow of fluid to and from a surgical handpiece.

EP-A-0,769,304 discloses a respiratory apparatus having the features of the preamble of claim 1. The present invention is characterized by the features of the characterizing portion of claim 1. Optional features are recited in the dependent claims.

The present invention provides a respiratory apparatus having greater utilisation of the carbon dioxide absorber.

Advantageous developments of the invention emerge from the sub-claims.

The advantage of the invention essentially consists in the fact that a carbon dioxide absorber fitted on the respiratory apparatus has a transponder with a memory chip, on which a specification of the employed absorbent and the current consumption state is stored. A transponder-interrogator provided on the respiratory apparatus on the one hand reads out data from the memory chip and calculates from the operational data of the respiratory apparatus an updated consumption state which is stored back into the memory chip. Stand-by phases, in which no absorption or only slight absorption of carbon dioxide is taking place, or the duration of artificial respiration can for example be detected as operational data. Since the updated consumption state is stored back onto the memory chip, the depletion of the absorbent can also be ascertained when the carbon dioxide absorber is connected to another respiratory apparatus. The product life of the carbon dioxide absorber can thus be retraced and carbon dioxide absorbers which are overlaid and no longer fully fit for use can be identified. Apart from the current consumption state, it is expedient to store on the memory chip the absorber type, the type of respiratory lime used, the production date and the expiry date.

It is advantageous also to store of a producer code on the memory chip. The producer code is also detected by the transponder-interrogator and compared in an analysing unit of the respiratory apparatus with a list of admissible producer codes. In the case of compliance, a release signal enabling the operation of the respiratory apparatus is generated. The effect of this is that only appliances from authorised suppliers can be connected to the respiratory apparatus.
To advantage, the information on the memory chip is analysed only when the connection element of the carbon dioxide absorber is completely locked home into the rapid-action coupling of the respiratory apparatus. For this purpose, the antenna and the transmitting power of the transponder-interrogator are designed in such a way that the contents of the memory chip can only be read out when the carbon dioxide absorber is locked home. A mechanical coding can also be present between the connection element and the rapid-action coupling, in order that the absorber can be introduced only in a preferential position in order to reduce interface tolerances.

Information concerning the operation of the carbon dioxide absorber is expediently ascertained from the respiratory gas flow through the carbon dioxide absorber and from the carbon dioxide concentration on the approach side of the carbon dioxide absorber. The employed respiratory mode can also be included in the calculation of the operational data. The degree of absorber depletion can be ascertained in the analysing unit from the operational data and the specification of the employed respiratory lime, such as type and lime volume, and displayed on a display unit in the form of percentage absorber depletion. A simplified display of the absorber depletion can be achieved with a light-emitting diode. A green light-emitting diode shows that the carbon dioxide absorber is ready for operation. A yellow light-emitting diode, on the other hand, signals impending absorber depletion and a red light-emitting diode is activated when the carbon dioxide absorber is no longer fit for use.

An example of embodiment of the invention will be explained in greater detail below with reference to the accompanying Figures, of which:
Figure 1 is a schematic diagram of a respiratory apparatus with a carbon dioxide absorber,
Figure 2 is a schematic diagram of the respiratory apparatus according to figure 1 with a locked-home carbon dioxide absorber.

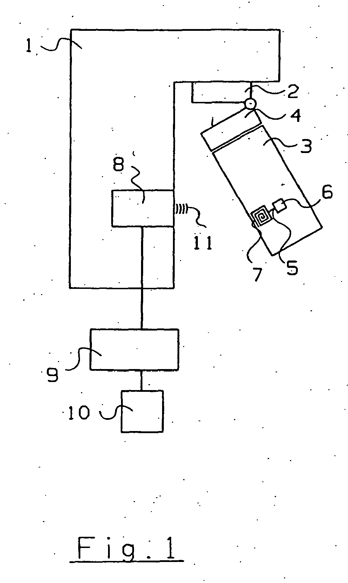

Figure 1 shows diagrammatically a respiratory apparatus 1 with a rapid-action coupling 2 for the connection of a carbon dioxide absorber 3. Carbon dioxide absorber 3 has a connection element 4 complementary to rapid-action coupling 2, said connection element being locked home into rapid-action coupling 2. A transponder 5 with a memory chip 6 and an antenna 7 is located on carbon dioxide absorber 3. Fixed on respiratory apparatus 1 is a transponder-interrogator 8, which is connected to an analysing unit 9 and a display unit 10.

Transponder-interrogator 8 can both read data on memory chip 6 and write updated data onto memory chip 6. Dashes 11 illustrate the range of transponder-interrogator 8.

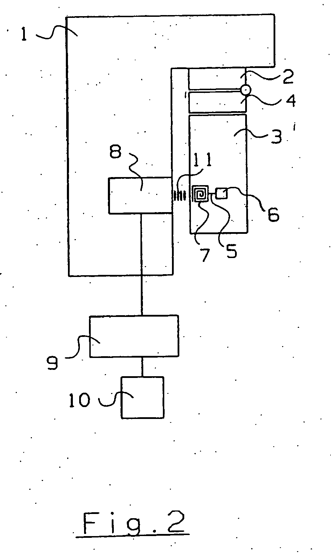

Figure 2 shows respiratory apparatus 1 with carbon dioxide absorber 3 completely locked home in rapid-action coupling 2. Here, antenna 7 of transponder 5 passes into the range of transponder-interrogator 8.

### The respiratory apparatus according to the invention operates in the following manner:

When carbon dioxide absorber 3 is delivered, the absorber type, the type of respiratory lime used, a producer code, the date of manufacturer, the expiry date and the consumption state are stored on memory chip 6 of the transponder. The consumption state, i.e. the absorber depletion, is 0% in the as-delivered state and the period for use of the respiratory lime lies between the date of manufacture and of the expiry date. Connection element 4 of carbon dioxide absorber 3 is introduced into rapid-action coupling 2 and locks home by swivelling, in accordance with figures 1 and 2. Antenna 7 of transponder 5 passes here into the reading range of transponder-interrogator 8. The data on memory chip 6 are then first read out into analysing unit 9. In a first step, the producer code is compared there with a list of approved producer codes. After a positive outcome, a check is made to see whether the expiry date has not yet been passed and a release signal enabling the operation of respiratory apparatus 1 is then generated.

Analysing unit 9 is constantly receiving from sensors (not shown in detail) information concerning the respiratory gas flow through carbon dioxide absorber 3, the carbon dioxide concentration on the approach side of carbon dioxide absorber 3 and the set respiratory mode. Analysing unit 9 calculates therefrom an updated consumption state, e.g. 20%, and stores this back into computer chip 6 together with an apparatus code of respiratory apparatus 1. The current consumption state of carbon dioxide absorber 3 is outputted in the form of a bar graph display via display unit 10. The product life of carbon dioxide absorber 3 can be monitored without interruption using the data on memory chip 6.

## Claims

1. A respiratory apparatus including:
a rapid-action coupling (2); and
a carbon dioxide absorber (3) with a connection element (4) complementary to the rapid-action coupling (2); the apparatus being **characterized by**:
a transponder (5) on the carbon dioxide absorber (3);
a memory chip (6) assigned to the transponder (5), which memory chip contains information concerning type, period for use and current consumption state of the absorbent; and
a transponder-interrogator (8) on the respiratory apparatus (1), which reads out data from the memory chip (6) and stores back into the memory chip (6) information acquired in the operation in the form of an updated consumption state.

2. The respiratory apparatus according to claim 1, in which a display unit (10) which reproduces the updated consumption state in the form of absorber depletion.

3. The respiratory apparatus according to claim 1 or 2, in which a producer code is also stored on the memory chip (6) and that the received producer code is compared with a list of approved producer codes in an analysing unit (9) and, upon agreement, a release signal enabling the operation of the respiratory apparatus (1) is generated.

4. The respiratory apparatus according to any one of claims 1 to 3, in which the information on the memory chip (6) is analysed only when the connection element (4) is completely locked home into the rapid-action coupling (9).

5. The respiratory apparatus according to any one of claims 1 to 4, in which information concerning the operation is ascertained from the respiratory gas flow through the carbon dioxide absorber and from the carbon dioxide concentration on the approach side of the carbon dioxide absorber (3).

## Patentansprüche

1. Beatmungsvorrichtung umfassend,
eine Schnellkupplung (2),
und einen Kohlendioxidabsorber (3) mit einem zur Schnellkupplung (2) komplementären Anschlusselement (4),
einen Transponder (5) am Kohlendioxidabsorber (3),
ein dem Transponder (5) zugeordneten Speicherchip (6), welcher Informationen über Typ, Verwendungszeitraum und aktuellen Verbrauchszustand des Absorptionsmittels enthält, und
ein Transponder-Abfragegerät (8) an der Beatmungsvorrichtung (1), welches Daten aus dem Speicherchip (6) ausliest und Informationen, die im Betrieb gewonnen wurden, in Form eines aktualisierten Verbrauchszustandes in den Speicherchip (6) zurückspeichert.

2. Beatmungsvorrichtung nach Anspruch 1, **gekennzeichnet durch** eine Anzeigeeinheit (10), welche den aktualisierten Verbrauchszustand in Form einer Absorbererschöpfung wiedergibt.

3. Beatmungsvorrichtung nach Anspruch 1 oder 2, **dadurch gekennzeichnet, dass** auf dem Speicherchip (6) zusätzlich ein Herstellercode gespeichert ist und dass in einer Auswerteeinheit (9) der empfangende Herstellercode mit einer Liste von zugelassenen Herstellercodes verglichen wird und bei Übereinstimmung ein den Betrieb der Beatmungsvorrichtung (1) ermöglichendes Freigabesignal erzeugt wird.

4. Beatmungsvorrichtung nach einem der Ansprüche 1 bis 3, **dadurch gekennzeichnet, dass** die Informationen des Speicherchips (6) nur dann ausgewertet werden, wenn das Anschlusselement (4) vollständig in die Schnellkupplung (9) eingerastet ist.

5. Beatmungsvorrichtung nach einem der Ansprüche 1 bis 4, **dadurch gekennzeichnet, dass** Informationen über den Betrieb aus dem Atemgasfluss durch den Kohlendioxidabsorber und der Kohlendioxidkonzentration anströmseitig des Kohlendioxidabsorbers (3) ermittelt werden.

## Revendications

1. Respirateur comprenant :
un raccord rapide (2) ; et
un absorbeur de dioxyde de carbone (3) comprenant un élément de connexion (4) complémentaire du raccord rapide (2) ; l'appareil étant **caractérisé par** :
un transpondeur (5) présent sur l'absorbeur de dioxyde de carbone (3) ;
une puce mémoire (6) associée au transpondeur (5), puce qui contient des informations concernant le type, la période d'utilisation et l'actuel taux de consommation d'absorbant ; et
un interrogeur de transpondeur (8) présent sur le respirateur (1), qui lit les données provenant de la puce mémoire (6) et remet en mémoire sur la puce (6) les informations récoltées lors de l'opération, sous forme d'état de consommation mis à jour.

2. Respirateur selon la revendication 1, dans lequel une unité d'affichage (10) reproduit l'état de consommation mis à jour sous forme de diminution de l'absorbant.

3. Respirateur selon la revendication 1 ou 2, dans lequel un code producteur est également stocké sur la puce mémoire (6) et le code producteur reçu est comparé à une liste de codes producteurs approuvée, présente dans une unité d'analyse (9) et, après accord, un signal de déclenchement permettant le fonctionnement du respirateur (1) est généré.

4. Respirateur selon l'une quelconque des revendications 1 à 3, dans lequel les informations présentes sur la puce mémoire (6) ne sont analysées que lorsque l'élément de connexion (4) est entièrement verrouillé dans le raccord rapide (9).

5. Respirateur selon l'une quelconque des revendications 1 à 4, dans lequel des informations concernant l'opération sont vérifiées, par l'écoulement de gaz respiratoire à travers l'absorbeur de dioxyde de carbone et par la concentration en dioxyde de carbone du côté arrivée de l'absorbeur de dioxyde de carbone (3).
